# EUROPÄISCHE PATENTANMELDUNG

(11) **EP 1 075 840 A2**
(43) Veröffentlichungstag der Anmeldung: **14.02.2001**
(21) Anmeldenummer: 00114354.4
(22) Anmeldetag: 05.07.2000
(51) Int. Cl.: A61K 38/57, A61P 35/00, A61P 37/00, A61P 31/18, A61P 25/28, A61P 9/00, A61P 29/00

(54) **Verwendung von Antithrombin III zur Prophylaxe und Therapie von Erkrankungen**

(30) Priorität: 13.08.1999 DE 19937656
(71) Anmelder: Aventis Behring GmbH, 35002 Marburg (DE)
(72) Erfinder: Römisch, Jürgen, Dr., 35041 Marburg (DE); Stauss, Harald, 35232 Dautphetal (DE)

(57) **Zusammenfassung**

Es wird die Verwendung von Antithrombin III zur Prophylaxe und Therapie von onkologischen und mit Neovaskularisierungen einhergehenden Erkrankungen, Immunkomplex-vermittelten und Autoimmun-Erkrankungen, viralen Infektionen, fibrosierenden und granulomatösen Erkrankungen, allergischen Erkrankungen, degenerativen Erkrankungen des Nervensystems und Arteriosklerose sowie akut entzündlichen Erkrankungen und Traumen beschrieben.

## Beschreibung

Gegenstand der Erfindung ist die Verwendung von Antithrombin III zur Prophylaxe und Therapie von Erkrankungen.

Es ist bekannt, dass Antithrombin III (AT) einer der wichtigsten Inhibitoren des Plasmas ist. Als Serin-Protease-Inhibitor reguliert AT III Reaktionen des Gerinnungssystems. Durch seine Eigenschaft, Heparin und mit diesem verwandte Substanzen zu binden, vermittelt es sowohl die gerinnungshemmende Wirkung solcher Antikoagulanzien als auch einen protektiven Effekt des Endothels und der darunter liegenden Matrix, die dem Heparin strukturell ähnliche Glykosaminoglykane wie Heparansulfat tragen. Die Aktivierung des AT III durch diese Kofaktoren führt zu einer Beschleunigung der Reaktion mit aktivierten Proteasen, vor allen mit denen der Gerinnungskaskade.

Die Eigenschaft, an Heparin zu binden, wird häufig auch dazu genutzt, AT III bspw. aus Plasma anzureichern und entsprechende Konzentrate zur prophylaktischen und therapeutischen Verwendung herzustellen. Besonders Patienten mit angeborenen oder erworbenen Mangelzuständen an AT III werden mit entsprechenden Produkten behandelt. Ein signifikanter Mangel an AT III, besonders während Erkrankungen wie der Sepsis, mit der häufig eine disseminierte, intravasale Gerinnung (DIC), verbunden mit einem Multiorganversagen und Schock, einhergehen, führt oft zum Tode. Neben der die Hämostase regulierenden Funktion des AT III wurden auch schon Eigenschaften beschrieben, die eine enzündungshemmende Wirkung nahelegen (1). Diese Hypothese basiert auf dem Befund der durch AT III vermittelten Freisetzung von Prostacyclin aus Endothelzellen, welches u.a. die Aggregation von Blutplättchen hemmt. Darüber hinaus wurde in in vitro Versuchen gezeigt, dass AT III die durch bakterielle Lipopolysaccharide stimulierte Freisetzung des als pro-inflammatorisch angesehenen Zytokins Interleukin-6 und die Expression des Gewebethromboplastins (Tissue factor) von Monozyten und Endothelzellen reduzieren kann. In Tiermodellen zeigte die Applikation von AT III signifikante, prophylaktische und therapeutische Effekte auf die Mortalität bei induzierter Sepsis und DIC. Außerdem wurden die Reperfusionsschäden nach künstlicher beigefügter Ischämie durch Ligation von Organgefäßen vermindert.

Die durch AT III vermittelten Effekte können bisher durch dessen Interaktion mit seinen Zielproteasen erklärt werden, wobei lösliche und Zell- bzw. Matrixgebundene Glykosaminoglykane beteiligt sind.

Bei vielen Erkrankungen spielen zellvermittelte Prozesse, besonders unter Beteiligung von Leukozyten, eine entscheidende Rolle. Ein physiologisch sinnvolles Maß an aktivierten Leukozyten trägt zur Eindämmung von z.B. Infektionsherden bei. Ist diese Reaktion jedoch dysreguliert, kommt es zur Schädigung von Geweben, die letztendlich zum Organversagen und zum Tod führen können.

Die Wanderung der Leukozyten, auch als Migration bezeichnet, wird durch die Ausschüttung von sog. Chemokinen gefördert, die Leukozyten entlang eines entsprechenden Konzentrationsgradienten zum Zielgebiet, z.B. zu einer Gewebeläsion, locken. Dort angekommen befinden sich die Zellen in einem aktivierten Zustand und schütten Mediatioren aus, die weitere gewebeschädigende Reaktionen auslösen oder verstärken können. Chemokine, wie das Interleukin-8, interagieren dabei mit Zellmembran-ständigen Rezeptoren auf Leukozyten und anderen Zellen und lösen intrazelluläre Signalreaktionen aus, die die Zelle zur Mobilität und Synthese oder Ausschüttung proinflammatorischer Substanzen veranlassen.

Überraschenderweise wurde nun gefunden, dass AT III die Migration von Leukozyten, also Granulozyten, Eosinophilen, Basophilen und Lymphozyten/Monozyten bei Stimulierung bzw. Anlockung durch ein Chemokin wie Interleukin-8, reduzieren oder zumindest steuern kann. Die Leukozyten werden dabei in einer von der AT III-Konzentration abhängigen Weise dahingehend beeinflusst, auf ein Chemokin nicht oder abgeschwächt zu reagieren, was einer sog. heterologen Desaktivierung entspricht. Hieraus ergeben sich Anwendungsmöglichkeiten für den Einsatz von AT III bei einer Reihe von mit Entzündungsreaktionen verbundenen Erkrankungen.

Gegenstand der Erfindung ist deshalb die Anwendung von AT III zur Prophylaxe und Therapie von Erkrankungen, insbesondere von onkologischen und mit Neovaskularisierungen einhergehenden Erkrankungen, Immunkomplexvermittelten und Autoimmun-Erkrankungen, viralen Infektionen, fibrosierenden und granulomatösen Erkrankungen, allergischen Erkrankungen, degenerativen Erkrankungen des Nervensystems und Arteriosklerose sowie akkutentzündlichen Erkrankungen und Traumen.

Unter den erfindungsgemäß behandelbaren Immunkomplex-vermittelten Erkrankungen sind Vaskulitiden und Granulomerkrankungen besonders hervorzuheben. Bei den Autoimmun-Erkrankungen werden gute Behandlungserfolge bei systemischen Lupus Erythematodus (SLE), rheumatoider Arthritis (RA) und Pemphigus beobachtet. Auch virale Infektionen, insbesondere auch HIV, können erfindungsgemäß durch den Einsatz von AT III behandelt werden. Unter den allergischen Erkrankungen, die mit AT III therapiert werden können, sind Asthma bronchiale, Rhinitis, Conjunctivitis und Dermatiden vor allem zu nennen. Auch bei degenerativen Erkrankungen des Nervensystems, z.B. bei der Alzheimer'schen Erkrankung, werden mit AT III Behandlungserfolge beobachtet.

Die bisher mit AT III durchgeführten biochemischen und zellulären Untersuchungen legen den Schluss nahe, dass AT III mit einem oder mehreren zellulären Rezeptoren interagiert und zu einer Blockade der Chemokinrezeptoren, besonders der sog. CXC-Rezeptroen führt, die dann nicht mehr auf Chemokine wie Interleukin-8 reagieren können. Als Folge bleibt die chemotaktische Migration der Leukozyten auf diese Stimuli hin aus. Daraus lässt sich ein signifikanter in vivo Effekt im Sinne einer Reduktion der Ausprägung bestimmter Krankheiten ableiten.

Die bisher vorliegenden Ergebnisse wurden experimentell in ex vivo Versuchen gewonnen. Durch Inkubation von Granulozyten mit AT III und anschließender Anlockung durch Interleukin-8, wie sie in vitro in sog. Boyden-Kammern durchgeführt werden, zeigte sich eine signifikante Reduktion der Leukozytenmigration, aus der für den Fachmann vertraute, prophylaktische und therapeutische Effekte resultieren.

Es ist zwar bekannt, dass AT III neben den erwähnten angeborenen und erworbenen Mangelzuständen auch bereits zur Prophylaxe und Therapie bei Sepsis und DIC eingesetzt werden kann (1). Tierexperimentelle Daten legen außerdem die Verwendung zur Reduktion des Reperfusionsschadens nach Ischämie (2) oder bei Organtransplantationen nahe, deren Abstoßungsreaktionen deutlich vermindert wurden (3).

Erfindungsgemäß konnte nun gezeigt werden, dass die therapeutischen und prophylaktischen Anwendungsmöglichkeiten von AT III viel umfassender sind, wobei diese Ergebnisse sowohl mit aus Plasma gereinigtem als auch mit rekombinant oder transgen gewonnenem Antithrombin III oder dessen Mutanten oder davon abgeleiteten Peptiden gewonnen werden konnten.

Die mit AT III prophylaktisch oder therapeutisch behandelbaren Erkrankungen lassen sich einteilen in
1. akute zellvermittelte Entzündungsreaktionen und
2. chronische zellvermittelte Entzündungsreaktionen.

Insbesondere sind darunter zu verstehen:
- Urtikaria und Angioödem
- Asthma
- Lungenemphysem
- Pemphigus
- Vaskulitis
- Graft-Versus-Host Erkrankung
- granulomatöse Entzündungserkrankungen
- rheumatoide Arthritis
- systemischer Lupus Erythematosus
- Gicht
- neutrophile Dermatosen
- fibrosierende Lebererkrankungen
- entzündliche Neoplasien
- mit Neovaskularisierung assoziierte Erkrankungen
- virale Infektionen, deren Mechanismus des Endringens in die Zielzellen über Chemokinerezeptoren erfolgt, wie HIV
- neurodegenerative Krankheiten
- vaskuläre Entzündungen, z.B. Atherosklerose, welche mit einer Chemokin vermittelten Infiltration durch Leukozyten einhergeht.

In Abhängigkeit von der jeweiligen Entzündungsreaktion kann AT III intravenös, subkutan, intradermal/intramuskulär oder topisch eingesetzt werden.

Die Erfindung wird durch das folgende Beispiel näher erläutert.

### Beispiel:

Neutrophile Granulozyten wurden aus Vollblut gesunder Spender präpariert. Dazu wurden dem Fachmann vertraute Methoden, wie die Separation durch Zentrifugation in geeigneten Medien wie dem Ficoll, angewendet. Nach der anschließenden Lyse verbliebener Erythrozyten wurden die Granulozyten durch nochmalige Zentrifugation isoliert. Nach Bestimmung der Zellzahlen wurden die Granulozyten umgehend im Chemotaxis-Test untersucht.

Dieser Test wurde in sog. Boyden-Kammern durchgeführt. Prinzipiell beruht die Methode darauf, dass die Zellen in einem Zwei-Kompartimentensystem über einen Membranfilter hinweg durch Aufbau eines chemotaktischen Gradienten angelockt werden. Üblicherweise werden die Zellen dabei in die "obere" Kammer pipettiert, das Chemokin in die "untere" Kammer. Entlang des Gradienten dringen die Zellen in die Poren des Filters ein. Bei ausreichend langer Inkubation durchwandern die Zellen den Filter und werden an dessen Unterseite oder in der untere Kammer ausgezählt. In dem beschriebenen Versuch wurde jedoch die Zellzahl der in den Filter gewanderten Zellen nach Fixierung und Färbung mikroskopisch bestimmt. Der chemotaktische Index gibt dabei den Effekt des Chemoattraktans wieder, der sich aus dem Quotienten der Zellzahlen der durch das Chemokin veranlassten Wanderung und der ungerichteten Migration (Chemokinese) ergibt.

Zur Untersuchung des die Zellmigration modulierenden Effektes wurden die Granulozyten mit steigenden Konzentrationen von AT für 15 Min. bei 37 C inkubiert. Nach mehrfachem Waschen der Zellen wurden diese in den Chemotaxistest eingesetzt. Als Chemokine wurden IL-8 (1 nM) oder C5a (0,1 nM) verwendet. Nach 30 Min. Inkubation bei 37 C wurden die Filtereinsätze gewaschen, die Zellen fixiert, angefärbt und ausgezählt. Die Chemokinese (ohne Lockstoff) sowie die Chemotaxis mit AT III unbehandelten Zellen (Positivkontrolle) wurde ebenfalls bestimmt. Der chemotaktische Index wurde wie oben beschrieben ermittelt.

Jede Versuchsreihe wurde in 4 parallelen Ansätzen durchgeführt, wobei Zellen von jeweils 3 gesunden Spendern verwendet wurden.

Das Ergebnis wird in der folgenden Tabelle verdeutlicht:

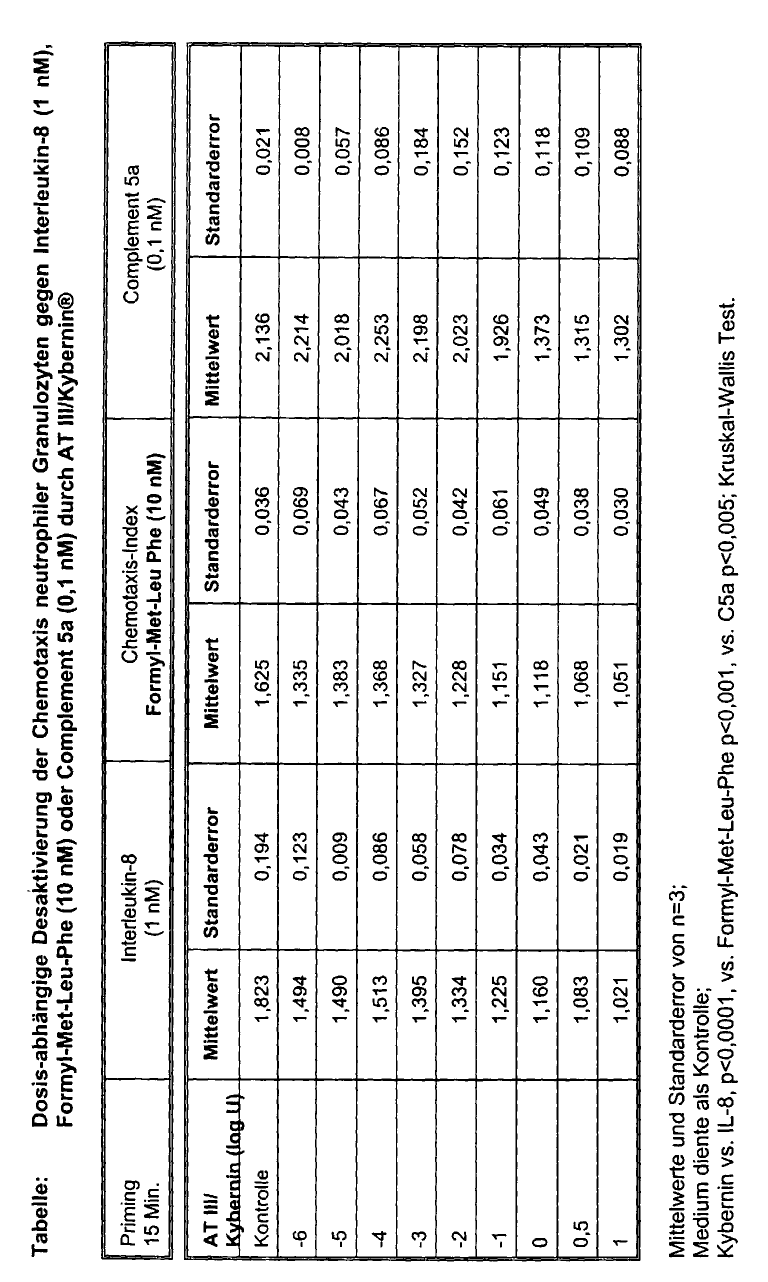

Die Tabelle verdeutlicht den durch AT III vermittelten Effekt auf die Granulozyten, der sich in einer von der AT III Konzentration abhängigen Weise darstellt. Gegenüber der Kontrolle, d.h. den nicht behandelten Zellen, reduziert sich die Anzahl der detektierbaren migrierten Zellen entsprechend. Daraus lässt sich schließen, dass die Granulozyten in ihrer Fähigkeit, entlang eines chemotaktischen Gradienten zu wandern, deutlich eingeschränkt bzw. inhibiert sind, woraus in vivo eine Verminderung der Krankheitsausprägung resultiert.

Equivalente Ergebnisse wurden auch mit anderen Leukozyten, wie Eosinophilen oder Monozyten erzielt.

### Literaturverzeichnis:

1. K. Okajima, Biomed. Progr. 9: 42-44 (1996).
2. Ostrovski, L.; Woodman, R.C.; Payne, D.; Teoh, D.; Ischemia/Reperfusion and Antithrombin III, Circulation Vol. 96, Nr. 7, 2302-2310 (1997).
3. Zuo, X.J., Okada, Y., Nicolaidou, E.; Toyoda, T.; Marchevsky, A.; Matloff, J.M. and Jordan, S.C.; Transplantation Proceedings, 31, 847-848 (1999).

## Patentansprüche

1. Verwendung von Antithrombin III zur Prophylaxe und Therapie von Erkrankungen, insbesondere von onkologischen und mit Neovaskularisierungen einhergehenden Erkrankungen, Immunkomplex-vermittelten und Autoimmun-Erkrankungen, viralen Infektionen, fibrosierenden und granulomatösen Erkrankungen, allergischen Erkrankungen, degenerativen Erkrankungen des Nervensystems und Arteriosklerose sowie akut entzündlichen Erkrankungen und Traumen.

2. Verwendung nach Anspruch 1, **dadurch gekennzeichnet**, dass Immunkomplex-vermittelte Krankheiten wie Vaskulitiden und Granulomerkrankungen behandelt werden.

3. Verwendung nach Anspruch 1, **dadurch gekennzeichnet**, dass Autoimmun-Erkrankungen wie systemischer Lupus Erythematosus, rheumatoide Arthritits und Pemphigus behandelt werden.

4. Verwendung nach Anspruch 1, **dadurch gekennzeichnet**, dass virale Infektionen, insbesondere HIV, behandelt werden.

5. Verwendung nach Anspruch 1, **dadurch gekennzeichnet**, dass allergische Erkrankungen wie Asthma bronchiale, Rhinitis, Conjunctivitis oder Dermatitiden behandelt werden.

6. Verwendung nach Anspruch 1, **dadurch gekennzeichnet**, dass degenerative Erkrankungen des Nervensystems wie die Alzheimer'sche Erkrankung behandelt werden.

7. Verwendung nach den Ansprüchen 1 bis 6, **dadurch gekennzeichnet**, dass aus Plasma oder rekombinant oder transgen exprimiertes Antithrombin III verwendet wird.

8. Verwendung nach den Ansprüchen 1 bis 7, **dadurch gekennzeichnet**, dass Antithrombin III intravenös, subcutan, intradermal, intramuskulär und topisch angewendet wird.
